(19) Europäisches Patentamt
European Patent Office
Office européen des brevets

(11) EP 0 861 595 A1

(12) EUROPEAN PATENT APPLICATION

(43) Date of publication:
**02.09.1998 Bulletin 1998/36**

(51) Int. Cl.$^6$: **A23F 3/34**, A23L 1/30

(21) Application number: **97103347.7**

(22) Date of filing: **28.02.1997**

(84) Designated Contracting States:
**DE FR GB**

(71) Applicant:
**Kowa Chemical Industries Co., Ltd.**
**Toyonaka-shi, Osaka 561 (JP)**

(72) Inventors:
- **Atsuchi, Mikito**
  **Ota-ku, Tokyo 146 (JP)**
- **Hirao, Yuri**
  **Ota-ku, Tokyo 144 (JP)**
- **Iwasaki, Yoshio**
  **Ota-ku, Tokyo 143 (JP)**

(74) Representative:
**Reinhard - Skuhra - Weise & Partner**
**Postfach 44 01 51**
**80750 München (DE)**

# (54) Gymnema inodorum roasted tea and method for preparing the same

(57) The invention relates to a *Gymnema inodorum* roasted tea having glucose absorption inhibiting action. The *Gymnema inodorum* roasted tea is obtainable by roasting dried *Gymnema inodorum* leaves at 150-200°C. The thus obtained *Gymnema inodorum* roasted tea retains its glucose absorption inhibiting substances, (3β,4α,16β)-16,23,28-trihydroxyolean-12-ene-3-yl-β-D-glucopyranuronic acid derivatives, and yet has moderate astringency, bitterness and a sweet and fragrant aroma. The *Gymnema inodorum* roasted tea of the invention is also rich in vitamins, amino acids and essential minerals necessary for the adjustment of biological functions.

FIG.1

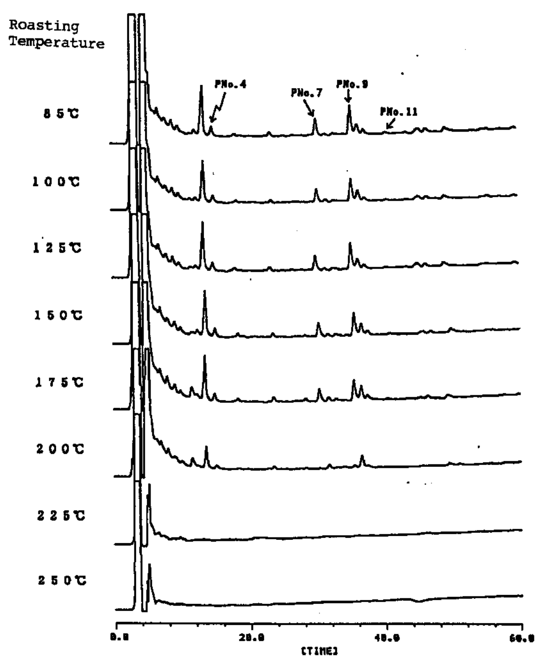


EP 0 861 595 A1

## Description

BACKGROUND OF THE INVENTION

1. Field of the Invention

The present invention relates to a *Gymnema inodorum* roasted tea obtainable by roasting leaves of a liana, *Gymnema inodorum*.

The present invention also relates to a health tea, food and drink containing the *Gymnema inodorum* roasted tea or fine powder thereof, and further relates to food and drink containing a decoction or a decoction extract of the *Gymnema inodorum* roasted tea.

2. Description of the Prior Art

Recently, the coming of a highly aged society and an increase in patients with adult diseases have become problems. With respect to diabetes, it is said that there is 5-6 millions of patients (including potential patients with hyperglycemia) in Japan. Non-insulin-dependent diabetes mellitus (NIDDM), pregnancy diabetes mellitus (GDM) and the like which are particularly high in incidence among various diabetes are considered to be caused mainly by overeating and corpulence (fatness). In particular, excessive intake of saccharides (such as sugar and starch) whose major component is glucose readily absorbed in intestinal tracts is said undesirable, because such excessive intake results in corpulence and puts a heavy burden upon β cells secreting insulin to thereby cause abnormalities in glucose tolerance.

Therefore, it is desirable to prevent excessive intake of those saccharides. However, appetite for food is one of human desires and it is extremely difficult to control one's appetite at present in the so-called "age of satiation". Under such circumstances, recently, there has been a growing interest in health teas which have an action of inhibiting the absorption of glucose generated by digestion of saccharides such as sugar and starch.

As a health tea having glucose absorption inhibiting action, there has been known *Gymnema sylvestre* tea. However, gymnemic acids which are the glucose absorption inhibiting components of this tea suppress sweet taste in taste buds and have bitterness. Therefore, these acids may damage the inherent flavor of food or drink and, thus, the conventional *Gymnema sylvestre* tea has many assignments to be solved for satisfying public taste as a favorite drink.

The present inventors have previously found that leaves of a liana, *Gymnema inodorum*, growing wild in Southeast Asia widely do not have gustatory modifying action such as suppression of astringency, bitterness or sweetness, and yet have an action to inhibit glucose absorption in intestinal tracts (Japanese Unexamined Patent Publication No. 3-172156). Then, the inventors have proposed a method for purifying a *Gymnema inodorum* leaf extract obtainable by extracting leaves of the above plant with a lower alcohol and/or water, and food and drink obtainable by adding the purified extract (Japanese Patent Application No. 6-319390).

Thereafter, the present inventors have further proposed some (3 β,4α,16β)-16,23,28-trihydroxyolean-12-ene-3-yl-β-D-glucopyranuronic acid derivatives, which are glucose absorption inhibiting substances contained in *Gymnema inodorum*, represented by general formula (I):

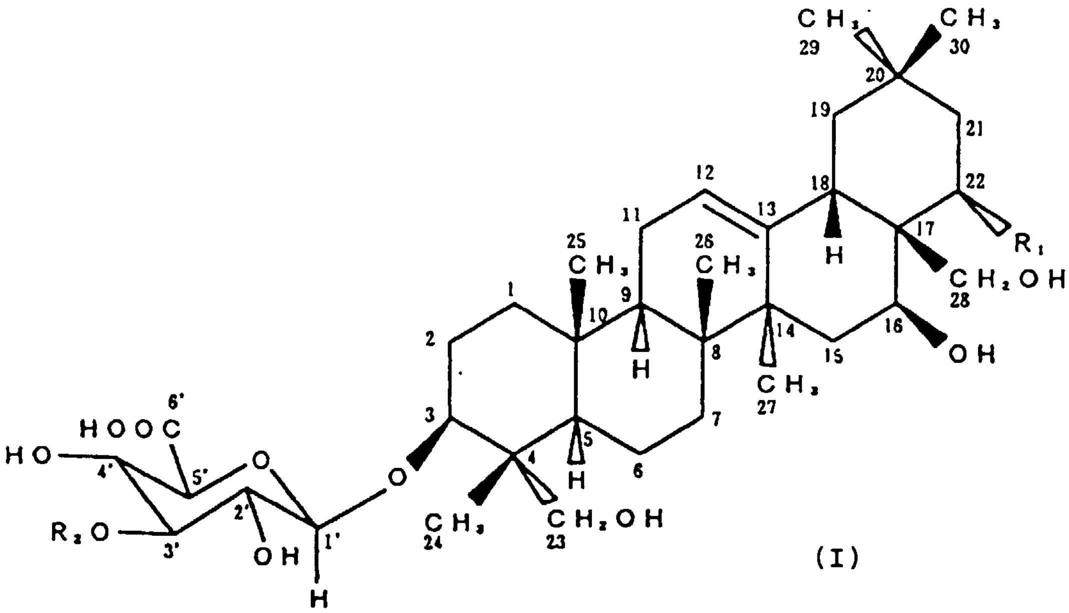

(I)

wherein $R_1$ is a hydrogen atom or a group represented by formula (II):

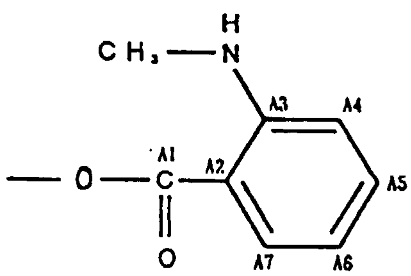

(II)

and $R_2$ is a hydrogen atom or a group represented by formula (III):

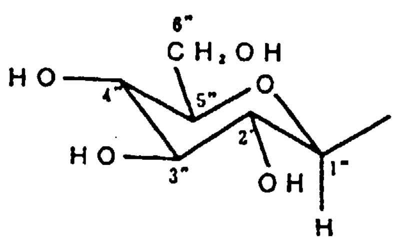

(III)

and a method for preparing the same.

OBJECTS AND SUMMARY OF THE INVENTION

It is an object of the present invention to process *Gymnema inodorum* leaves into an easy-to-drink health tea paying attention to the inherent properties of *Gymnema inodorum* leaves and extract thereof that they do not have gustatory modifying action such as suppression of astringency, bitterness or sweetness, and yet they have glucose

absorption inhibiting action. It is another object of the present invention to provide a health tea comprising the resultant *Gymnema inodorum* tea, food and drink containing a decoction of the tea, food and drink containing a decoction extract of the tea, food and drink containing fine powder of the tea, and the like.

The present inventors have made intensive and extensive researches on the use of *Gymnema inodorum* leaves containing the above-mentioned glucose absorption inhibiting substances as a health tea. As a result, it has been found that a *Gymnema inodorum* roasted tea obtainable by roasting *Gymnema inodorum* leaves under specific conditions has moderate astringency and bitterness, has a sweet and fragrant aroma, and yet retains the glucose absorption inhibiting action in intestinal tracts. Further, this tea has been found to be rich in vitamins, amino acids and minerals and, therefore, to be an excellent material as a health tea or a favorite drink. Thus, the present invention has been achieved.

The *Gymnema inodorum* roasted tea which is obtained by roasting *Gymnema inodorum* leaves at 150-200°C for 0.5-30 minutes has been found to retain their glucose absorption inhibiting substances, i.e., (3β,4α,16β)-16,23,28-trihydroxyolean-12-ene-3-yl-β-D-glucopyranuronic acid derivatives, vitamins, amino acids and the like, have moderate astringency and bitterness, and a sweet and fragrant aroma, and yet be rich in essential minerals necessary for the adjustment of biological functions.

(1) The invention relates to a *Gymnema inodorum* roasted tea obtainable by roasting *Gymnema inodorum* leaves.
(2) The invention relates to a *Gymnema inodorum* roasted tea comprising at least one (3β,4α,16β)-16,23,28-trihydroxyolean-12-ene-3-yl-β-D-glucopyranuronic acid derivative, which is a glucose absorption inhibiting substance, represented by general formula (I):

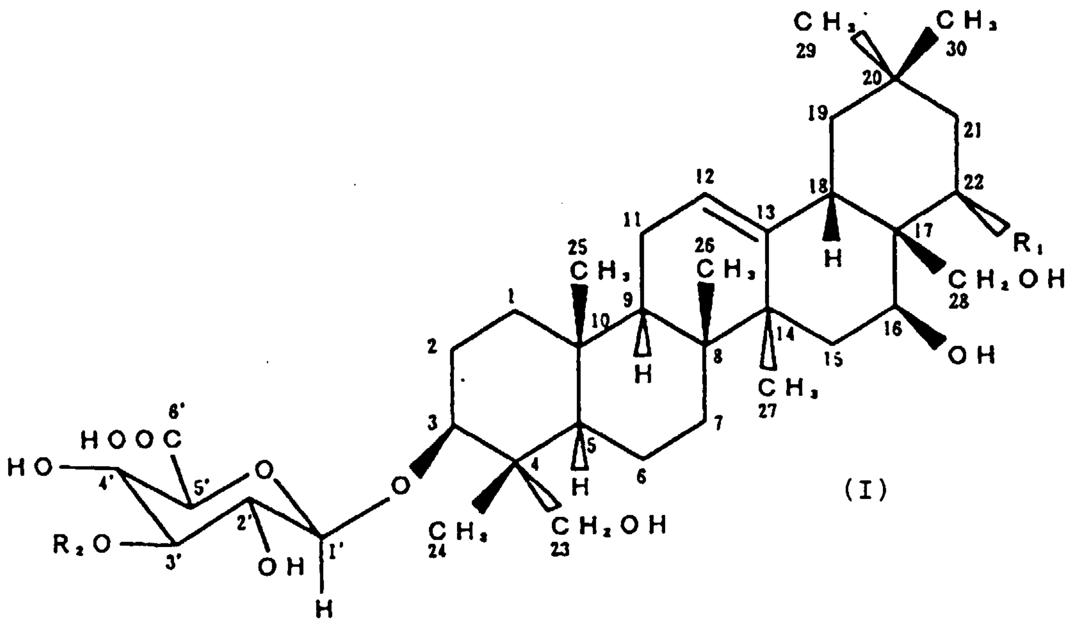

(I)

wherein $R_1$ is a hydrogen atom or a group represented by formula (II):

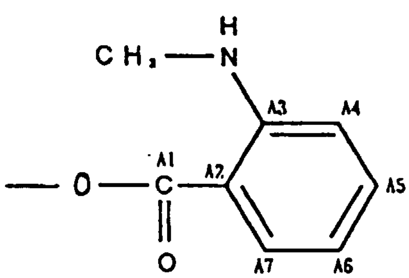

(II)

and $R_2$ is a hydrogen atom or a group represented by formula (III):

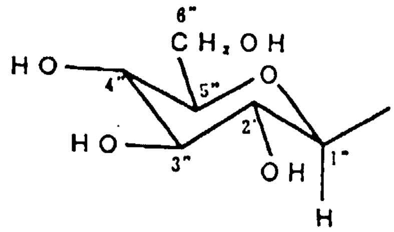

(III)

(3) The invention also relates to a method for preparing the *Gymnema inodorum* roasted tea of (1) or (2) above. More specifically, the invention relates to a method for preparing the tea comprising roasting dried *Gymnema inodorum* leaves at 150-200°C for 0.5-30 minutes.

(4) Further, the invention relates to a health tea containing the *Gymnema inodorum* roasted tea of (1) or (2) above alone or in combination with other tea.

(5) Further, the invention relates to a food or drink containing a decoction of the *Gymnema inodorum* roasted tea of (1) or (2) above.

(6) Further, the invention relates to a food or drink containing a decoction extract of the *Gymnema inodorum* roasted tea of (1) or (2) above.

(7) Further, the invention relates to a food or drink containing fine powder of the *Gymnema inodorum* roasted tea of (1) or (2) above.

BRIEF DESCRIPTION OF THE DRAWINGS

Fig. 1 is an HPLC chart on the components of each of the *Gymnema inodorum* roasted teas obtained under specific roasting temperature conditions described in the Example.

Fig. 2 is a graph showing the relationships between the roasting temperatures used in the Example and the contents of glucose absorption inhibiting substances in each of the *Gymnema inodorum* roasted teas obtained.

Fig. 3 is a graph showing the relationships between the roasting temperatures used in the Example and the suitability as a drink of each of the *Gymnema inodorum* roasted teas obtained.

Fig. 4 is a graph showing the relationships between the roasting temperatures used in the Example and the glucose absorption inhibiting action (as determined by a glucose tolerance test) of each of the *Gymnema inodorum* roasted teas obtained.

DETAILED DESCRIPTION OF THE INVENTION

Hereinbelow, a method for preparing the *Gymnema inodorum* roasted tea of the invention will be described in detail.

Drying Step

*Gymnema inodorum* leaves picked are dried at 50-90°C until its moisture content becomes about 5% using a carrier drier, tray drier or ventilating hand drier.

Cutting Step

Then, in order to achieve uniform roasting, dried *Gymnema inodorum* leaves are cut into small pieces 5-10 mm square before roasting using a roll cutter or screen cutter.

Roasting Step

Subsequently, using a far-infrared ray rotary roaster, infrared ray rotary roaster, rotary ventilating drier, automatic continuous tea toasting machine or sieve toaster, the dried *Gymnema inodorum* leaves cut into squares are roasted while adjusting the heater so that the temperature of the dried leaves become 150-200°C, preferably 160-180°C for 0.5-30 minutes. Then, the leaves are immediately cooled with a cooling fan.

When the roasting temperature is 150°C or below, a decoction of the resultant tea presents an unpleasant grassy smell and thus is not desirable as a drink. When the roasting temperature is 200°C or above, a decoction of the resultant tea presents severe bitterness, and the glucose absorption inhibiting function of the leaves is lost since the glucose absorption inhibiting substances, (3β,4α,16β)-16,23,28-trihydroxyolean-12-ene-3-yl-β-D-glucopyranuronic acid derivatives are decomposed by heat.

Cutting Step

Then, the roasted and cooled *Gymnema inodorum* tea is cut further into smaller pieces 2-5 mm square using a roll cutter or screen cutter.

Sieving/Selecting Step

Using a horizontal sieve, an air separator and the like, large leaves or yellow leaves and powder etc. are removed from the roasted, cooled and cut *Gymnema inodorum* tea to thereby obtain a *Gymnema inodorum* roasted tea 2-5 mm square.

In the *Gymnema inodorum* roasted tea of the invention, the glucose absorption inhibiting substances, (3β,4α,16β)-16,23,28-trihydroxyolean-12-ene-3-yl-β-D-glucopyranuronic acid derivatives, have not been lost. Thus, the *Gymnema inodorum* roasted tea of the invention is able to inhibit the absorption of glucose in intestinal tracts.

In addition, the *Gymnema inodorum* roasted tea of the invention has moderate astringency and bitterness, and a sweet and fragrant aroma and, thus, is easy to drink. Besides, this tea is rich in vitamins, minerals, amino acids and the like, and is preferable as a health tea or a favorite drink.

With respect to a method for decocting the *Gymnema inodorum* roasted tea of the invention, it is preferable to use hot water of 70-100°C in an amount of 0.5-2.0 liters, preferably 0.7-1.5 liters per 10 g of tea leaves, and preferably the leaves are decocted for 0.5-3 minutes. The resultant decoction may be cooled in a refrigerator or the like to be taken as iced tea.

With respect to a manner of drinking the *Gymnema inodorum* roasted tea of the invention, it is most effective to drink about 1 to 3 cups of the tea before each meal and between meals in view of its glucose absorption inhibiting action. Of course, one may drink this tea any time as one's favorite drink.

The *Gymnema inodorum* roasted tea of the invention or a decoction thereof can be used not only by itself but also in a mixture with other tea or a decoction thereof as a health tea or health drink. In addition, the *Gymnema inodorum* roasted tea of the invention or a decoction thereof can be mixed with various foods and food materials to prepare health foods. Examples of materials which may be mixed with the *Gymnema inodorum* roasted tea or a decoction thereof to prepare a health tea or health drink include the following materials.

Adzuki bean, acerola, *Gynostemma pentaphyllum*, alfalfa, chestnut, fig leaves, ginkgo leaves, turmeric tea, oolong tea, oriental senna, seed coat of plantain, barley, orange peel, Japanese persimmon leaves, licorice, chrysanthemum flowers, *Aloe arborescens* Mill. var. *natalensis* Berg., guavalong tea, guava, Chinese box thorn, *Sasa veitichii*, sesame, black soybean, mulberry leaf tea, brown rice tea, black tea, *Panax schinseng* leaves, saffron, hawthorn, *Cortinellus shiitake*, perilla leaves, jasmine tea, dietary fibers, field horsetail, buckwheat, soybean, chicory, evening primrose, *Hyndrangea strigosa* or *H. umbellata* tea, *Houttuynia cordata* tea, *Eucommia ulmoides* Olivev. tea, maize seeds, Indian millet, the base of sea tangle, lotus leaves, naked barley, Job's tears, *Lagerstroemia speciosa* tea, *Cassia obtusifoli*a tea, *Cassia minosoides* tea, barnyard millet, loquat leaves, yunnan tea, *Plantago ovata,* safflower, pine needles, mate tea, barley tea, *Acer nikoense* tea, mugwort tea, mangosteen fruit, green tea, rooibos tea, *Fomes japonicus*, lemongrass, and decoctions thereof.

Examples of materials which may be mixed with a decoction of the *Gymnema inodorum* roasted tea or an extract of such a decoction to prepare a health food or health drink include the following materials.

Almond, *Agaricus*, chives, *Angelica utilis* Makino leaves, adzuki bean, acerola, avocado, hydrangea tea seasoned with crude salt, *Gynostemma pentaphyllum*, alfalfa, *Aloe barbadensis*, turmeric, eels, plantain, olive oil, seaweeds, oyster shell powder, oyster, pollen, cod-liver oil, chrysanthemum flowers, fish oils, savory, chitin/chitosan, guava, guar gum, gardenia, cumin seeds, glucomannan, gluten, black vinegar, chlorella, bay leaves, coriander seeds, bone meal, sesame, sesame oil, sea tangle, comfrey, sage, Japanese pepper, *Cortinellus shiitake,* corb shell, perilla leaves, ginger, ginger roots, dietary fibers and snapping turtle.

Plum, *Mentha piperita,* buckwheat syrup, soybean, *Zizyphi Fructus,* thyme, dandelion, boiled and dried juvenile sardines, evening primrose, evening primrose oil, *Panax pseudoginseng* Wall. var. *notoginseng,* garden marigold, vegetative wasp, *Houttuynia cordata, Passiflora caerulea*, dried small sardines, carrot, garlic, the base of sea tangle, the sarcocarp of Japanese apricot, honey, Job's tears, *Cassia torosa,* common guava, *Hizikia fusiforme, Hyssopus officinalis*, caraway, blueberry, plum, propolis, safflower oil, spinach, hop, pine resin, mate, majoram, green mussel, basil, salt made by burning seaweeds, melissa, Japanese flowering quince, mulukhiya, yam roots, mangosteen fruit,

lavender, egg yolk oil, logan fruit extract, green tea extract, apple vinegar, rooibos, liver, lemongrass, lotus, rose hip, rosemary, lovage, and extracts thereof.

Irish moss extract, red clover extract, kamila tree (monkey-face tree) extract, *Solidago japonica* Kitam. extract, glue obtained from ass skin, *Acacia catechu* extract, anise seed extract, *Althaea officinalis* root extract, *Polygonatum odoratum* Druce var. *japonicum* Hara rhizome extract, ginkgo leaf extract, catnip extract, fennel extract, turmeric extract, *Quercus salicina* extract, *Acanthopanax senticosus* extract, *Plectranthi Herba* extract, *Polygonatum macrantum* root extract, *Panax schinseng* extract, *Hypericum erectum* Thunb. extract, olive leaf extract, Japanese persimmon leaf extract, zedoary extract, valerian extract (including valerian roots), and caffein.

German chamomile extract, guarana extract, *Trichosanthes cucumeroides* seeds, licorice extract, colts foot leaves extract, Chinese bellflower extract, *Aloe arborescens* Mill. var. *natalensis* Berg. extract, *Lonicera japonica* extract, *Veronica sibirica* extract, Chinese box thorn seed extract, Chinese box thorn leaf extract, *Sasa veitichii* extract, common vervain extract, *Orthosiphon spiralis*, *Galium odoratum* extract, chlorophyll, cassia bark extract, *Cassia obtusifolia* seed extract, *Acanthopanacis Radicis Cortex* extract, cobra extract, *Trigonella foenum-graecum* seed extract, saffron extract, hawthorn extract, gardenia fruit extract, *Panax pseudoginseng* Wall. var. *notoginseng* extract, *Cornus officinalis* extract, *Zizyphus jujuba* Mill. seed extract, *Kaempferiae Rhizoma* extract, *Nomame Herba* extract, *Adenophora triphylla* DC. subsp. *aperticampanulata* Kitam. root extract, plantain seed extract, plantain (whole plant) extract, *Houttuyniae Herba* extract, madder extract, *Crataegus oxyacantha* seed extract, European elder extract and yarrow extract.

Elder extract, *Curculigo orchioides* rhizome extract, mulberry leaf extract, star anise extract, *Lophatherum gracile* extract, clove extract, *Margaritifera* extract, orange peel extract, bitter orange peel extract, *Juniperus rigida* fruit extract, reindeer's horn extract, nutmeg extract, Japanese honeysuckle leaf extract, *Trimeresurus flavoviridis* extract, *Glehnia littoralis* extract, mustard seed extract, *Mylabris* extract, *Agkistrodon* acutus extract, leech extract, betel nut palm extract, charred globe fish extract, *Plantago ovata* extract, buchu leaves extract, safflower extract, long-plantain extract, dandelion root extract, linden tree flower extract, boldo leaves extract, oyster shell extract, pine needle extract, *Agkistrodon halys* extract, *Polygonum aviculare* extract, *Actinidia polygama* extract, field horsetail extract, gum tree extract, *Rumex japonicus* Houtt. root extract, tansy extract, *Fomes japonicus* extract, ground ivy extract, lotus leaf extract, and *Anthemis nobiles* extract.

Amino acids, sodium benzoate, EPA • DHA, *Plantago ovata* seeds, nucleic acids (DNA/RNA), various embryos, various vitamins, calcium, β-caroten, brown sugar, black vinegar enzyme, yeast, powdered mash of pure rice vinegar, *Spirulina*, lactic acid bacteria, lactose, Job's tears enzyme, *Lactobacillus bifidus*, glucose, soybean milk powder, lecithin, royal jelly, etc.

Examples of common foods to which a decoction extract or powder of the *Gymnema inodorum* roasted tea may be added include the following foods.

Margarine, shortening, alcoholic drinks (Japanese sake, synthetic sake, low-class distilled spirits, beer, fruit wine, whisky, brandy, spirits, liqueur, miscellaneous alcohols, sweet sake used as seasoning), cooling drinks [soda, fruit drink, frozen fruit drink, mineral (spring) water, etc.], powdered cooling drinks, fish meat sausage, fish meat ham, ground fish meat, fish meat paste, meat products (ham, sausage, bacon, corned beef, etc.), noodles (raw noodle, boiled noodle, dried noodle, instant noodle), soybean cakes (soybean cake, processed soybean cake), jams (jam, preserve, marmalade), flour paste, soy sauce, sauces (sauce, puree, ketchup, mayonnaise, dressing), soybean paste, vinegars (brewed vinegar, synthetic vinegar), fresh cakes (Japanese unbaked cakes, buns with a bean-jam filling, cakes, other buns), confectionery [baked confectionery, rice cracker, cookie & biscuit, candy, chocolate, chewing gum, buns (excluding unbaked ones), doughnut, cheap sweets, sweets made of beans, candied fruit, etc.], sherbet, sugar, fish flour to be sprinkled on cooked rice, etc.

## PREFERRED EMBODIMENTS OF THE INVENTION

Hereinbelow, the present invention will be described in more detail with reference to the following Example, which should not be construed as limiting the scope of the present invention.

## EXAMPLE

### Drying Step

*Gymnema inodorum* new buds (2000 kg) picked in July in Chiang Mai district, Thailand were dried at 80°C for 3 hours until the moisture content reached 4.7% using a tray drier to thereby obtain 48.6 kg of dried *Gymnema inodorum* leaves.

Cutting Step

Then, the 48.6 kg of dried *Gymnema inodorum* leaves were cut into small pieces 5-10 mm square using a screen cutter.

Roasting Step

Using a far-infrared ray rotary roaster, 5 kg of the dried and cut *Gymnema inodorum* leaves were roasted while adjusting the heater to so that the temperature of the dried leaves became 85°C for 1.2 minutes (rate of rotation in the rotary furnace: 45 rpm), and then the leaves were immediately cooled with a cooling fan.

Then, eight 5 kg samples of the dried *Gymnema inodorum* leaves were roasted in a similar manner for the same roasting period (1.2 minutes) at roasting temperatures of 100, 125, 150, 175, 200, 225 and 250°C, respectively, and cooled in the same manner.

Cutting Step

Then, each of the 8 kinds of roasted and cooled *Gymnema inodorum* teas was cut into smaller pieces 2-5 mm square using a screen cutter.

Sieving/Selecting Step

Using a horizontal sieve, large leaves or powder etc. were removed from each of the 8 kinds of *Gymnema inodorum* roasted teas to thereby obtain 8 kinds of *Gymnema inodorum* roasted teas 2-5 mm square.

The contents of glucose absorption inhibiting substances, (3β,4 α,16β)-16,23,28-trihydroxyolean-12-ene-3-yl-β-D-glucopyranuronic acid derivatives, contained in each of the 8 kinds of *Gymnema inodorum* roasted teas were quantitatively analyzed by high performance liquid chromatography (HPLC) under the following conditions. (HPLC Quantitative Analysis Conditions)

| | |
|---|---|
| Pretreatment of samples: | 10 g of each of the 8 *Gymnema inodorum* roasted teas obtained in the Example was extracted with 100 ml of 50% ethanol for 2 hours at room temperature, and the extraction residue was extracted with fresh another 100 ml of 50% ethanol for 2 hours at room temperature. The resultant two extracts were mixed and used as a sample. |
| Amount of sample loaded: | 100 μl |
| Column: | TSKgel ODS-80$T_M$ (reversed-phase distribution system/Tosoh Corp.) |
| Column size: | 4.6 mm I.D. x 25 cm |
| Eluent: | Solution A Acetonitrile:distilled water:acetic acid = 40.0:60.0:0.1 (v/v%)<br>Solution B Acetonitrile:distilled water:acetic acid = 60.0:40.0:0.1 (v/v%)<br>Solution C Acetonitrile:distilled water:acetic acid = 90.0:10.0:0.1 (v/v%) |
| Gradient: | 0→ 60 min. A→ B linear gradient<br>60 → 66 min. B→ C linear gradient<br>66 → 120 min. isocratic elution with Solution C |
| Flow rate: | 0.8 ml/min. |
| Detector: | Ultraviolet-visible detector (210 nm) |
| Column temperature: | 40°C |

The HPLC chart is shown in Fig. 1. The results of quantitative analysis based on the HPLC analysis are shown in Table 1 and Fig. 2. In the Table and Figures, "PNo. 4" represents a glucose absorption inhibiting substance, (3β,4α,16β)-16,23,28-trihydroxyolean-12-ene-3-yl-β-D-glucopyranuronic acid; "PNo. 7" represents a glucose absorption inhibiting substance, (3β,4α,16β,22α)-22-(N-methylanthraniloxy)-16,23,28-trihydroxyolean-12-ene-3-yl-3-O-β-D-glucopyranosyl-β-D-glucopyranuronic acid; "PNo. 9" represents a glucose absorption inhibiting substance, (3β,4α,16β,22α)-22-(N-methylanthraniloxy)-16,23,28-trihydroxyolean-12-ene-3-yl-β-D- glucopyranuronic acid; "PNo. 11" represents a glucose absorption inhibiting substance (the chemical formula and scientific name of this substance are unknown, but this substance has been confirmed to have an N-methylanthraniloxy group from the UV and IR spectrum and is presumed to have a chemical structure similar to those of the components PNo.7 and PNo. 9).

Table 1

| Contents of Glucose Absorption Inhibiting Substances in Various *Gymnema inodorum* Roasted Teas (in 100 g of leaves) | | | | | |
|---|---|---|---|---|---|
| Roasting temperature (°C) | Content of Glucose Absorption Inhibiting Substance (mg/100g tea leaves) | | | | |
| | PNo. 4 | PNo. 7 | PNo. 9 | PNo. 11 | Total |
| 85 | 256 | 203 | 330 | 43 | 832 |
| 100 | 248 | 191 | 315 | 28 | 782 |
| 125 | 240 | 179 | 300 | 27 | 746 |
| 150 | 228 | 176 | 267 | 25 | 696 |
| 175 | 210 | 166 | 244 | 25 | 645 |
| 200 | 98 | 10 | 27 | 11 | 146 |
| 225 | 0 | 0 | 0 | 6 | 6 |
| 250 | 0 | 0 | 0 | 0 | 0 |

As is clear from Table 1 and Fig. 2 *infra*, it has been found that 4 glucose absorption inhibiting substances, (3β,4α,16β)-16,23,28-trihydroxyolean-12-ene-3-yl-β-D-glucopyranuronic acid derivatives, undergo thermal decomposition at a roasting temperature of 200°C or above and that they are almost lost if treated at such a temperature for only about 1 minute. Accordingly, it is presumed that the glucose absorption inhibiting action inherent to *Gymnema inodorum* leaves has been also lost in tea leaves treated at such a temperature.

A functional (sensory) test was conducted on the 8 kinds of *Gymnema inodorum* roasted tea obtained in the Example using, as panelists, staffs of the laboratory to which the present inventors belong (age 22-60; male and female; total 10 persons) for astringency, bitterness, sweetness, aroma and color, and overall impression (synthetic evaluation).

The evaluation in the functional test was conducted by 6-stage evaluation from 0 to 5 points. The evaluation standards were as follows; 5: very good; 4: good; 3: slightly good 2: slightly bad; 1: bad; and 0:very bad. The decoction of *Gymnema inodorum* roasted tea used in the functional test was prepared by decocting 10 g of tea leaves of each *Gymnema inodorum* roasted tea in 1.0 liter of hot water at 85°C for 2 minutes.

The results of the above test are shown in Table 2 and Fig. 3 *infra*.

Table 2

| Results of the Functional Test on Astringency, Bitterness, Aroma and Color (Average value from 10 persons) | | | | | | | |
|---|---|---|---|---|---|---|---|
| Roasting temp. (°C) | Test Items | | | | | Synthetic evaluation | Remarks |
| | Astringency | Bitterness | Sweetness | Aroma | Color | | |
| 85 | 2.8 | 2.5 | 1.9 | 1.5 | 3.3 | 1.7 | Grassy smell<br>Yellow green |
| 100 | 2.9 | 2.4 | 1.9 | 1.7 | 3.3 | 1.7 | Grassy smell<br>Yellow green |
| 125 | 3.3 | 2.8 | 2.8 | 2.2 | 2.7 | 2.7 | Slightly grassy<br>Light amber |
| 150 | 3.5 | 3.4 | 3.2 | 3.5 | 3.6 | 3.7 | Fragrant<br>Amber |
| 175 | 3.9 | 4.1 | 3.3 | 4.0 | 3.8 | 4.1 | Fragrant<br>Tasty |

Table 2 (continued)

| Results of the Functional Test on Astringency, Bitterness, Aroma and Color (Average value from 10 persons) | | | | | | | |
|---|---|---|---|---|---|---|---|
| Roasting temp. (°C) | Test Items | | | | | Synthetic evaluation | Remarks |
| | Astringency | Bitterness | Sweetness | Aroma | Color | | |
| 200 | 3.0 | 3.4 | 2.7 | 2.7 | 3.1 | 3.1 | Slightly bitter<br>Light amber |
| 225 | 2.3 | 2.5 | 1.4 | 1.7 | 0 | 1.4 | Bitter<br>Light in color |
| 250 | 1.5 | 2.5 | 0.8 | 0.9 | 0 | 0 | Bitter<br>Light in color |

As is clear from Table 2 and Fig. 3 *infra*, decoctions of *Gymnema inodorum* tea roasted at 150°C or below are yellow green in color as in green tea but have an unpleasant grassy smell and are weak in aroma. Thus, *Gymnema inodorum* tea roasted at 150°C or below has been found not suitable to serve as a drink.

Hereinbelow, the glucose absorption inhibiting action of the 8 kinds of *Gymnema inodorum* roasted tea obtained in the Example will be described with reference to the results of a glucose tolerance test.

The details of the method of the glucose tolerance test were as follows.

[Method of the glucose tolerance test]

Preparation of samples:
10 g of each of the 8 *Gymnema inodorum* roasted teas obtained in the Example was decocted with 1.0 liter of hot water at 85°C for 2 minutes and then filtered. The filtrate was concentrated to 10 ml to obtain a sample.
Experiment animal:
7-week old male SD rats (purchased from Nippon S.L.C.) were used after one-week preparatory breeding in which the rats were fed with a solid feed (F-2 for mouse/rat) and water in a breeding chamber at 23-26°C under a humidity of 50-60%.
Dose of the sample:
2,500 μl/kg
Dose of glucose:
glucose 2,000 mg/kg
Preparation of glucose:
The glucose to be administered was made into an aqueous solution containing 200 mg of glucose per ml of water.
Administration of the sample and the glucose solution:
The sample and the glucose solution were administered orally to rats compulsorily using an oral sound. The control group was administered the medium of the sample and the glucose solution.
Volume of administration:
The sample was administered at a dose of 2.5 ml/kg and the aqueous glucose solution was administered at a dose of 10 ml/kg.
Glucose tolerance test:
5 rats weighing 250-265 g after undergoing a fast of 16 hours (free access to water was allowed) were used as one group. One hour after the removal of the water container from each cage, blood samples were collected from rats and the amount of blood sugar was determined for each sample. Rats were selected so that average blood sugar values in individual groups were equal. Then, the sample was administered and, immediately after that, glucose was administered orally. 15, 30, 60 and 120 minutes after the glucose loading, a blood sample was collected from each rat. Each time, a sample was taken from the tail vein by 50 μl, mixed with 50 μl of 10 mg/ml sodium fluoride (dissolved in saline) and then used for determination. The amount of blood sugar in whole blood was determined using an automatic glucose determination apparatus (Model GA-1120 manufactured by Kyoto Daiichi Kagaku).
Statistical analysis:
The results of the test were shown in average values. Average values and standard errors were calculated, and the significant difference between the control group and each treatment group was tested. Dunnett's multiple comparison test was used as a test method and the significant level was set at less than 5% in the ratio of risk.

The results are shown in Table 3 and Fig. 4 *infra.*

In Fig. 4, the value for the blood sugar increase inhibiting ratio $In_{30}$ (%) was calculated by the following formula:

$$In_{30}(\%) = 100 - [(Bi_{30} - Bi_0)/(Bc_{30} - Bc_0)] \times 100$$

wherein

$In_{30}$ (%): blood sugar increase inhibiting ratio (%) 30 minutes after the glucose loading;
$Bi_{30}$: blood sugar value (mg/dl) in a test sample 30 minutes after the glucose loading;
$Bi_0$: blood sugar value (mg/dl) in a test sample before the glucose loading;
$Bc_{30}$: blood sugar value (mg/dl) in the control group 30 minutes after the glucose loading; and
$Bc_0$: blood sugar value (mg/dl) in the control group before the glucose loading.

Table 3

| Glucose Absorption Inhibiting Action in Various *Gymnema inodorum* Roasted Teas (Results of Glucose Tolerance Test) | | | | | | |
|---|---|---|---|---|---|---|
| Roasting temp. (°C) | Dose (µl/kg, p.o.) | Blood Sugar Value (mg/dl) | | | | |
| | | Before loading | Minutes after Glucose Loading | | | |
| | | | 15 | 30 | 60 | 120 |
| Control G. | - | 63±1 | 118±8 | 136±4 | 112±2 | 81±3 |
| 85 | 2500 | 65±3 | 87±4* | 98±3* | 107±3 | 94±4 |
| 100 | 2500 | 63±3 | 88±2* | 98±2* | 97±3 | 95±3 |
| 125 | 2500 | 63±2 | 91±3* | 96±1* | 99±2 | 95±1 |
| 150 | 2500 | 66±3 | 96±5* | 100±4* | 100±2 | 95±2 |
| 175 | 2500 | 65±3 | 93±4* | 103±3* | 112±1 | 102±6 |
| 200 | 2500 | 66±3 | 97±3* | 116±4* | 109±2 | 90±2 |
| 225 | 2500 | 66±2 | 130±6 | 131±6 | 109±4 | 83±2 |
| 250 | 2500 | 66±2 | 125±5 | 136±3 | 110±3 | 85±3 |

In the above Table, mark "*" indicates that there is a significant difference ($p<0.05$) in statistical analysis.

As is clear from Table 3 and Fig. 4 *infra,* it has been found that those *Gymnema inodorum* teas which have been roasted at 200°C or above and lost the 4 glucose absorption inhibiting substances, (3β,4α,16β)-16,23,28-trihydroxyolean-12-ene-3-yl-β-D-glucopyranuronic acid derivatives no longer possess their glucose absorption inhibiting action.

In other words, in the relationship between the roasting temperature and the contents of glucose absorption inhibiting substances, or in the relationship between the roasting temperature and the glucose absorption inhibiting action seen in the glucose tolerance test, it has been found that those *Gymnema inodorum* teas which have been roasted at 200°C or above no longer possess their glucose absorption inhibition action since their glucose absorption inhibiting substances have been lost by thermal decomposition. In addition, in the relationship between the roasting temperature and the results of the functional test, it has been found that decoctions of those *Gymnema inodorum* teas roasted at 150 °C or below have an unpleasant grassy smell and, thus, are not suitable as a drink. Accordingly, an appropriate roasting temperature for the *Gymnema inodorum* roasted tea ranges from 150 to 200°C.

Table 4 below shows the contents of vitamins, essential amino acids, minerals and caffein in a *Gymnema inodorum* tea roasted at 175 °C for 1.2 minutes.

Table 4.

Nutrients Contained in *Gymnema inodorum* Roasted Tea

(Roasted at 175℃)

| Nutrient | Content | Method of Analysis |
|---|---|---|
| [Basic components] | | |
| Moisture | 7.1% | Normal pressure heat-drying method |
| Protein | 23.4% | Kjeldahl method |
| Lipid | 3.9% | Soxhlet extraction method |
| Fiber | 12.9% | Improved Henneberg-Stohmann method |
| Ash content | 12.8% | Direct ashing method |
| Sugar | 37.5% | |
| Anhydrous caffein | 0.002% | HPLC |
| Tannin | 2.40% | Folin-Denis method |
| [Vitamins] | | |
| $\beta$-carotene | 6.09 mg/100 g | HPLC |
| Vitamin $B_1$ | 0.11 mg/100 g | HPLC |
| Vitamin $B_2$ | 1.10 mg/100 g | HPLC |
| Vitamin C | 42 mg/100 g | HPLC |
| Vitamin E | 36.5 mg/100 g | HPLC |
| $\alpha$-tocopherol | 13.2 mg/100 g | |
| $\beta$-tocopherol | 18.5 mg/100 g | |
| $\gamma$-tocopherol | 0.9 mg/100 g | |
| $\delta$-tocopherol | 3.9 mg/100 g | |
| Nicotinic acid | 8.75 mg/100 g | Microbiological assay |
| [Essential amino acids] | | |
| Lysine | 30 mg/100 g | Automatic amino acid analysis |
| Phenylalanine | 26 mg/100 g | |
| Leucine | 33 mg/100 g | |

| | | |
|---|---|---|
| Isoleucine | 28 mg/100 g | |
| Methionine | Not detected | |
| Valine | 60 mg/100 g | |
| Threonine | Not detected | |
| Tryptophan | 17 mg/100 g | |
| [Minerals] | | |
| Phosphorus | 394 mg/100 g | Vanadomolybdic acid absorption spectrophotometry |
| Iron | 59.8 mg/100 g | o-Phenanthroline absorption spectrophotometry |
| Calcium | 1.57% | Potassium permanganate volumetric analysis |
| Sodium | 11.6 mg/100 g | Atomic absorption spectrometry |
| Potassium | 4.01 % | Atomic absorption spectrometry |
| Magnesium | 544 mg/100 g | Atomic absorption spectrometry |
| Copper | 7.00 ppm | Atomic absorption spectrometry |
| Zinc | 34.2 ppm | Atomic absorption spectrometry |
| Manganese | 163 ppm | Atomic absorption spectrometry |
| Nickel | 2.37 ppm | Atomic absorption spectrometry |
| Selenium | 0.19 ppm | Fluorescence photometry |
| Aluminium | 432 ppm | Atomic absorption spectrometry |
| Boron | 52 ppm | ICP emission spectrochemical analysis |

It has been found that a *Gymnema inodorum* roasted tea obtainable by roasting dried *Gymnema inodorum* leaves at 150-200°C for 0.5-30 minutes is retaining its glucose absorption inhibiting substances, (3β,4α,16β)-16,23,28-trihydroxyolean-12-ene-3-yl-β-D-glucopyranuronic acid derivatives, vitamins and amino acids and yet possesses moderate astringency, bitterness and a sweet and fragrant aroma, and that this *Gymnema inodorum* roasted tea is rich in essential minerals necessary for the adjustment of biological functions and contains only an extremely small amount of caffein.

EFFECT OF THE INVENTION

According to the present invention, it is possible to process *Gymnema inodorum* leaves into a tea without losing or damaging the glucose absorption inhibiting substances contained therein. The *Gymnema inodorum* roasted tea obtained by the method of the invention has glucose absorption inhibiting action in intestinal tracts, has moderate astringency, bitterness and a sweet and fragrant aroma and yet is rich in vitamins, essential amino acids and essential minerals. In other words, according to the present invention, it is possible to provide an extremely easy-to-drink health tea of *Gymnema inodorum* which does not have gustation modifying action such as suppression of astringency, bitterness or sweetness, but has glucose absorption inhibiting action; a health tea obtainable by combining the *Gymnema inodorum* tea; food and drink containing a decoction extract of the *Gymnema inodorum* tea; and food and drink containing fine powder of the *Gymnema inodorum* tea.

**Claims**

1. A *Gymnema inodorum* roasted tea obtainable by roasting *Gymnema inodorum* leaves.

2. A *Gymnema inodorum* roasted tea comprising at least one (3β,4α,16β)-16,23,28-trihydroxyolean-12-ene-3-yl-β-D-glucopyranuronic acid derivative, which is a glucose absorption inhibiting substance, represented by general formula (I):

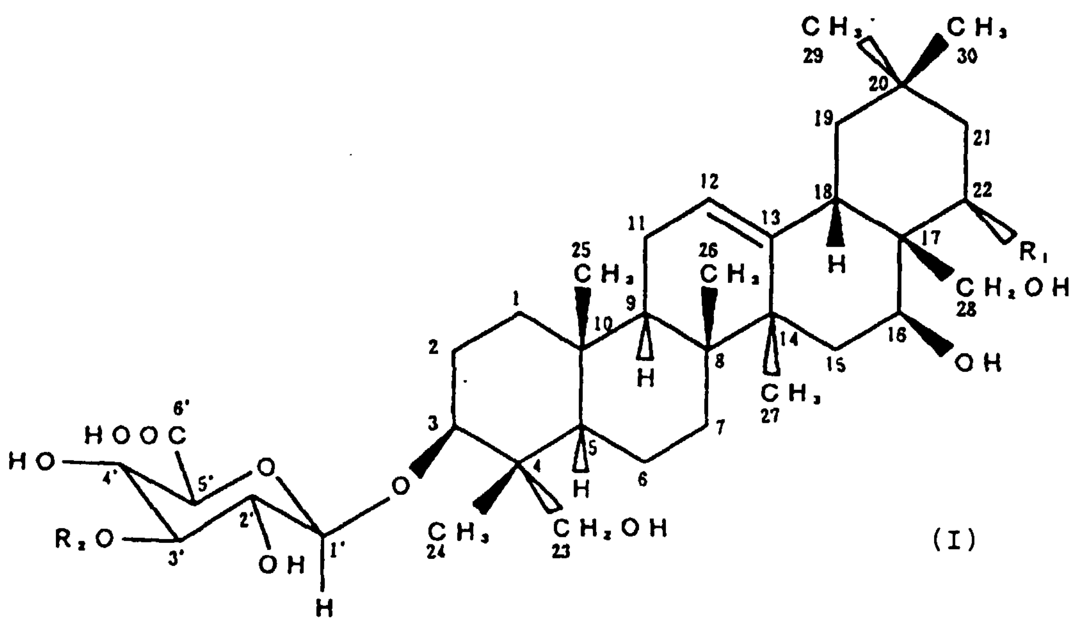

(I)

wherein $R_1$ is a hydrogen atom or a group represented by formula (II):

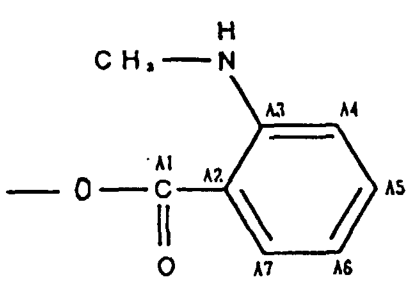

(II)

and $R_2$ is a hydrogen atom or a group represented by formula (III):

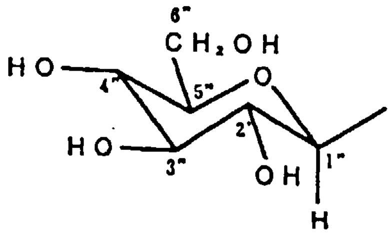

(III)

3. A method for preparing a *Gymnema inodorum* roasted tea, comprising roasting dried *Gymnema inodorum* leaves at 150-200°C for 0.5-30 minutes.

4. A health tea containing the *Gymnema inodorum* roasted tea of claim 1 or claim 2.

5. A food or drink containing a decoction of a *Gymnema inodorum* roasted tea.

6. A food or drink containing a decoction extract of a Gymnema inodorum roasted tea.

7. A food or drink containing fine powder of a *Gymnema inodorum* roasted tea.

FIG.1

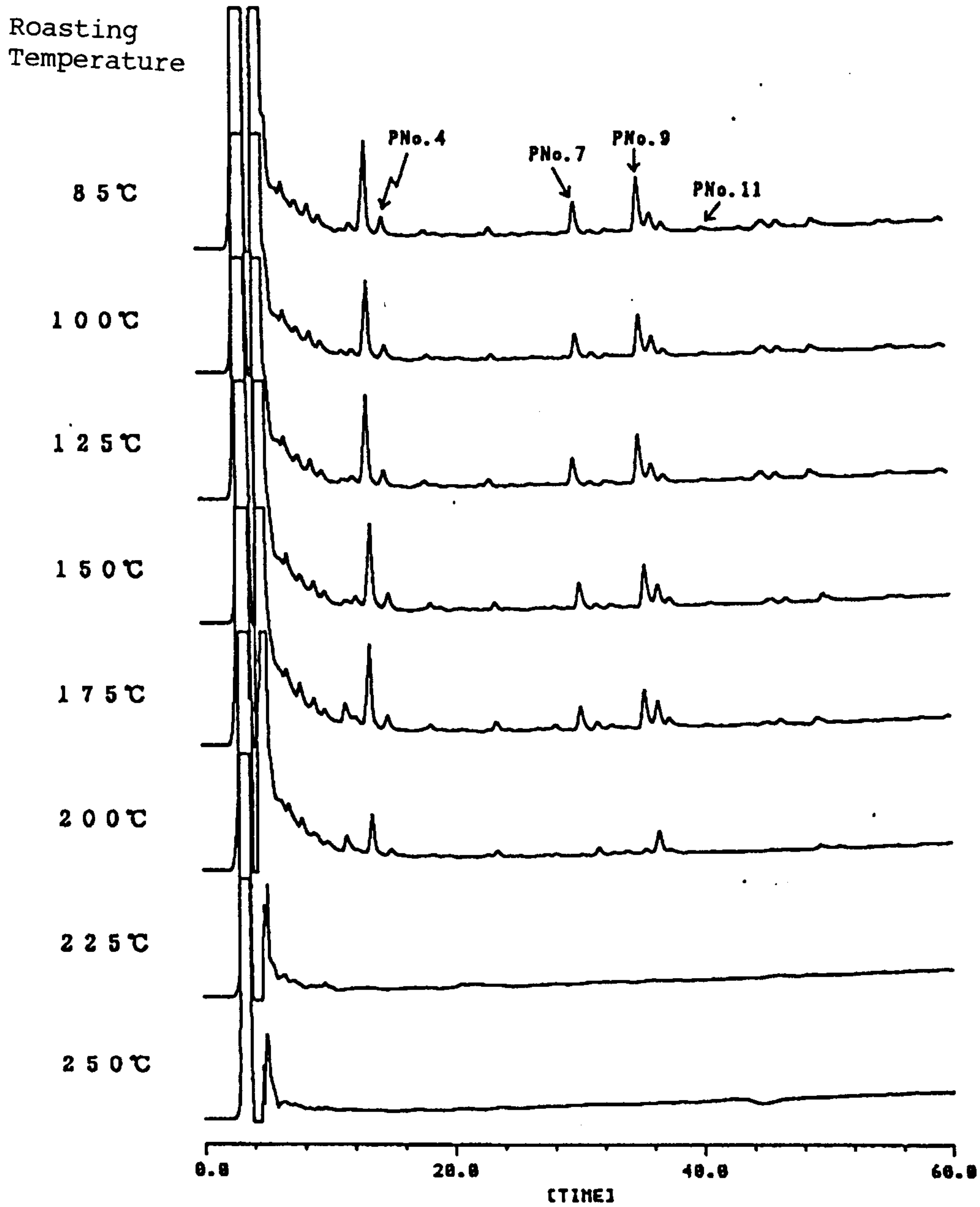
Roasting
Temperature
85℃
100℃
125℃
150℃
175℃
200℃
225℃
250℃
PNo.4
PNo.7
PNo.9
PNo.11
0.0
20.0
40.0
60.0
[TIME]

FIG.2

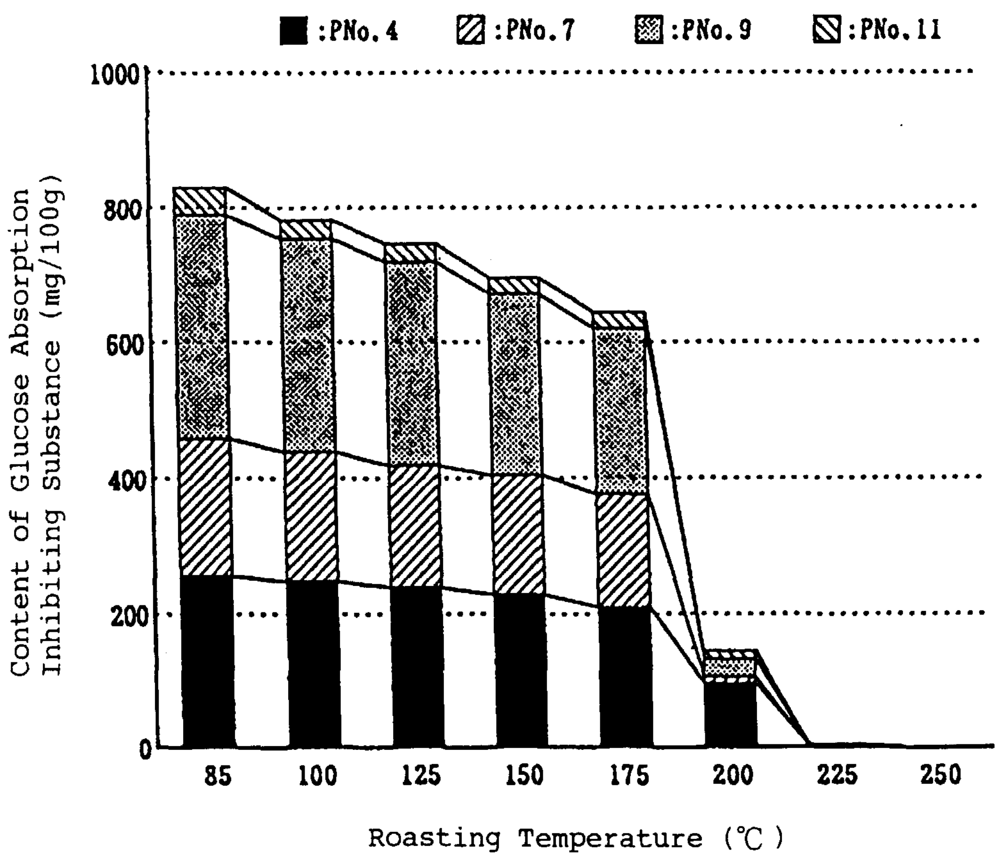
■:PNo.4
▨:PNo.7
▩:PNo.9
▧:PNo.11
1000
800
600
400
200
0
Content of Glucose Absorption Inhibiting Substance (mg/100g)
85
100
125
150
175
200
225
250
Roasting Temperature (℃)

FIG.3

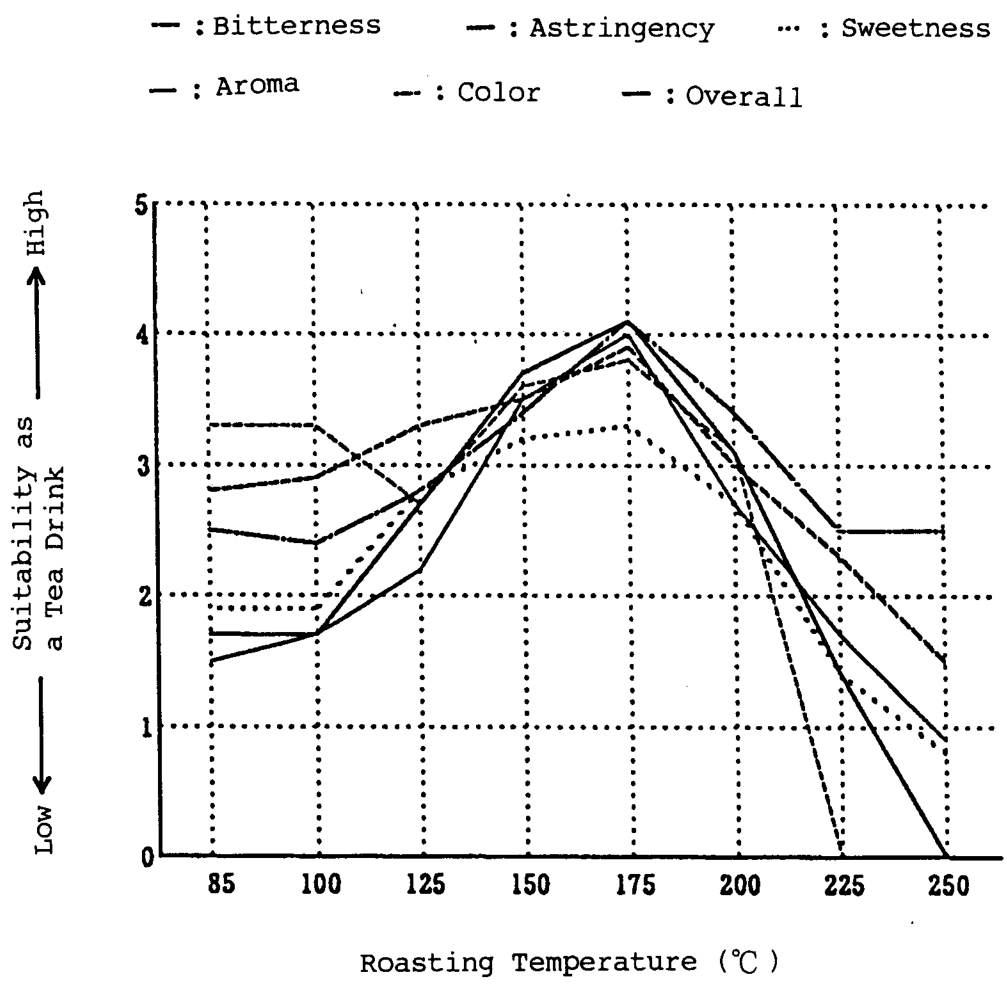
— : Bitterness
— : Astringency
··· : Sweetness
— : Aroma
—- : Color
— : Overall
5
4
3
2
1
0
Low
Suitability as a Tea Drink
High
85
100
125
150
175
200
225
250
Roasting Temperature (℃)

FIG.4

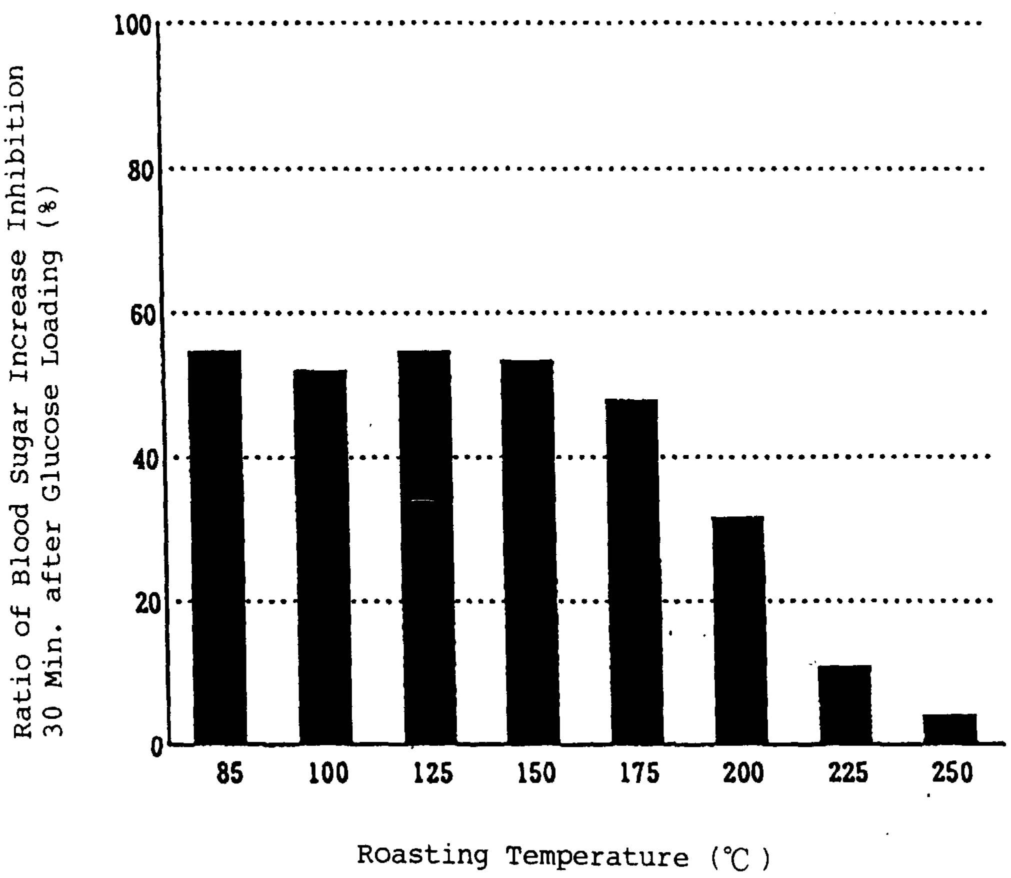
Ratio of Blood Sugar Increase Inhibition
30 Min. after Glucose Loading (%)
100
80
60
40
20
0
85
100
125
150
175
200
225
250
Roasting Temperature (℃)

European Patent Office

# EUROPEAN SEARCH REPORT

Application Number
EP 97 10 3347

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X<br>Y | EP 0 515 755 A (KOWA CHEMICAL INDUSTRIES)<br>* page 3, line 54 - page 4, line 20; claims 1,2 *<br>* page 1, line 44 - line 49 *<br>--- | 1-6<br>7 | A23F3/34<br>A23L1/30 |
| A | PATENT ABSTRACTS OF JAPAN<br>vol. 096, no. 012, 26 December 1996<br>& JP 08 208689 A (KOWA CHEM IND CO LTD)<br>* abstract *<br>--- | 1-7 | |
| Y | PATENT ABSTRACTS OF JAPAN<br>vol. 013, no. 079 (C-571), 22 February 1989<br>& JP 63 269950 A (ITOCHU SEITO KK;OTHERS: 01), 8 November 1988,<br>* abstract *<br>--- | 7 | |
| A | PATENT ABSTRACTS OF JAPAN<br>vol. 018, no. 012 (C-1150), 11 January 1994<br>& JP 05 252897 A (SEKISUI CHEM CO LTD), 5 October 1993,<br>* abstract *<br>--- | 1-7 | TECHNICAL FIELDS SEARCHED (Int.Cl.6)<br>A23F<br>A23L |
| A | PATENT ABSTRACTS OF JAPAN<br>vol. 015, no. 150 (C-0824), 16 April 1991<br>& JP 03 030653 A (ITOCHU SEITO KK), 8 February 1991,<br>* abstract *<br>----- | 1-7 | |

The present search report has been drawn up for all claims

| Place of search | Date of completion of the search | Examiner |
|---|---|---|
| THE HAGUE | 4 July 1997 | DESMEDT, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)